# EUROPEAN PATENT APPLICATION

(11) **EP 3 936 156 A1**
(43) Date of publication of application: **12.01.2022**
(21) Application number: 20201866.9
(22) Date of filing: 14.10.2020
(51) Int. Cl.: A61L 2/04, A61L 2/06, A61L 2/24, B08B 5/00, B08B 7/00, B08B 9/30, A23B 7/005, A23L 3/18, A23B 4/005

(54) **APPARATUS FOR EXPOSING OBJECTS TO A DISINFECTING HEAT TREATMENT AND METHOD USING SAID APPARATUS**

(30) Priority: 10.07.2020 ES 202031518 U
(71) Applicant: Torsa Sistemas S.L., 29590 Málaga (ES)
(72) Inventor: Santana Moreno, Raúl, 29590 Málaga (ES); Serrano Morales, Francisco Javier, 29590 Málaga (ES)
(74) Representative: Balder IP Law, S.L.

(57) **Abstract**

Apparatus (1) for exposing objects (O) to a disinfecting heat treatment comprising a chamber (H) provided with an inlet (11) and an outlet (12) for allowing the passage of a conveyor belt (B) for conveying the objects (O) and passing them through the chamber (H), a heat distribution system inside the chamber (H) having an air convection subsystem (S1) for maintaining a first minimum temperature (T1) in the chamber (H), a compressed air injection subsystem (S2) for injecting compressed air at a second temperature (T2), the subsystems (S1, S2) being configured such that the second temperature (T2) is higher than the first temperature (T1). The invention also relates to a method for disinfecting that uses the inventive apparatus.

## Description

### FIELD OF THE INVENTION

The present invention belongs to the field of devices for eliminating viruses and pathogens from objects by passing them through a chamber wherein heat treatment is applied to said objects.

### BACKGROUND OF THE INVENTION

Systems for the continuous disinfection of objects based on a chamber provided with an inlet and an outlet, both intended for allowing the passage of a conveyor belt for conveying the objects and passing them through the chamber, are known.

The arrangement of a UV ray source, a chemical-based sprayer, or a steam-based sprayer, inside the chamber is known.

However, these systems present drawbacks because, when performing disinfection, for example, to eliminate viruses and pathogens, the UV ray-based system may not reach all the spaces since these spaces must be within the field of view of the UV ray sources. Chemical-based systems present the obvious drawback of the need for consumables that must be replenished over time. Furthermore, not all products can be exposed to these chemicals. Steam-based systems are useful when moisture is required for cleaning purposes; however, it presents the drawback that not all objects can be treated with steam. For example, parcels consist mainly of cardboard, so this treatment is unsuitable.

Systems based on the use of steam are described in documents EP3048901, ES2288364A1 or US2006040029A, both intended for the food industry.

### DESCRIPTION OF THE INVENTION

To overcome the mentioned drawbacks, the present invention proposes an apparatus for exposing objects to a disinfecting heat treatment, the apparatus comprising a chamber provided with an inlet and an outlet, both intended for allowing the passage of a conveyor belt for conveying the objects and passing them through the chamber, a heat distribution system inside the chamber, characterised in that the heat distribution system comprises:
- an air convection subsystem for maintaining a first minimum temperature in the chamber;
- a compressed air injection subsystem for injecting compressed air at a second temperature;
the subsystems being configured such that the second temperature is higher than the first temperature.

The invention is therefore based on passing the objects to be disinfected into a volume or chamber where a temperature sufficient to eliminate viruses or pathogens that may be found on the surfaces of the objects is maintained by means of the first subsystem. As it is air-based, all the exposed areas of the products are subjected to air at a first temperature. Having said that, in addition to the treatment provided by the first subsystem, the invention contemplates adding a treatment by means of a second subsystem of hot compressed air oriented towards the object, at a temperature higher than the convection temperature, to drastically reduce or eliminate any surviving virus or pathogen.

This arrangement by means of two subsystems having different ways of supplying heat allow the compressed air to be highly effective, since the surface of the object, as well as the air in the chamber are already at a high temperature, such that the injected hot air does not suffer such a pronounced temperature drop which would occur in the absence of the first subsystem.

An advantage of the hot air is that it can be used only as long as necessary so as to not affect the object O, or only on the surface thereof where pathogens or viruses can be found as the result of the prior handling thereof. It is difficult to carry out this control when water steam is used due to its high latent heat and to the fact that it may have much more thermal inertia. In contrast, gusts of hot air cause highly localised heating in the space and in time, which prevent damaging the objects.

In some embodiments:
- the first subsystem comprises at least one fan and a resistor;
- the second subsystem comprises a compressed air source and air injection nozzles connected to the source by means of a distribution circuit, the circuit being provided with electrical resistors for heating the air to the second temperature.

Therefore, the present invention relates to a system that only requires a compressed air source and an electrical power source.

In some embodiments, the second subsystem comprises collectors each provided with several nozzles. Preferably, the nozzles are arranged such that they produce an air curtain or air blade. Therefore, treatment of the entire surface of the object with the high-temperature injected air will be ensured when passing through the curtain.

In some embodiments, the apparatus comprises a valve, preferably a solenoid valve, arranged between the compressed air source and the air heating means.

In some embodiments, the apparatus comprises one or several temperature sensors for detecting the temperature of the injected air and/or of the chamber. In this manner, it can be ensured that both the temperature of the convection temperature bath and the air injected with the nozzles are at the suitable temperature, and therefore the operating conditions of the apparatus can be adjusted depending on the environmental conditions and on the objects to be treated.

In some embodiments, the apparatus comprises a code reader arranged before the inlet, such that the objects can be identified and characterised to adjust the air heating parameters.

In some embodiments, the first subsystem is sized to maintain a minimum temperature of 80 °C in the chamber.

In some embodiments, the second subsystem is configured for increasing the temperature of the air coming from the compressed air source to a temperature higher than 140 °C.

In some embodiments, the air from the compressed air source is at a pressure comprised between 4 and 6 bar, and is preferably 5 bar.

In some embodiments, the apparatus comprises insulating curtains in the inlet and in the outlet of the chamber, the insulating curtains preferably being movable Teflon belts.

In some embodiments, the apparatus comprises a controller for controlling fan control relays, heating resistors and valves.

Lastly, in some embodiments, the apparatus comprises a screen for displaying the operation of the invention, the configuration of the parameters, or information about the treated objects such as, for example, the disinfection rate or the number of disinfected objects.

Further advantages and features of the invention will become apparent from the detailed description which follows and will be particularly indicated in the attached claims.

### BRIEF DESCRIPTION OF THE FIGURES

To complement the description and for the purpose of making the features of the invention more readily understandable according to a practical exemplary embodiment thereof, a set of figures is attached as an integral part of the description in which the following has been depicted with an illustrative and nonlimiting character:
Figure 1 is a block diagram of the first air heating subsystem of the apparatus according to the invention.
Figure 2 is a highly schematic drawing of the apparatus of the invention, coupled to a conveyor belt.
Figure 3 is a block diagram of the second air heating subsystem of the apparatus according to the invention.
Figure 4 is a block diagram of the control system of the apparatus of the invention, together with power components.

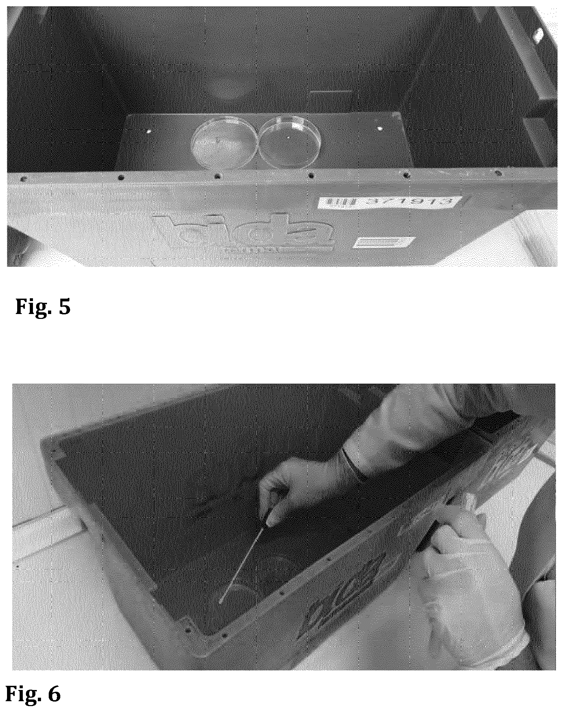

Figure 5 shows a pair of petri dishes with bacteria placed in the container used in the test for anti-viral effectiveness.

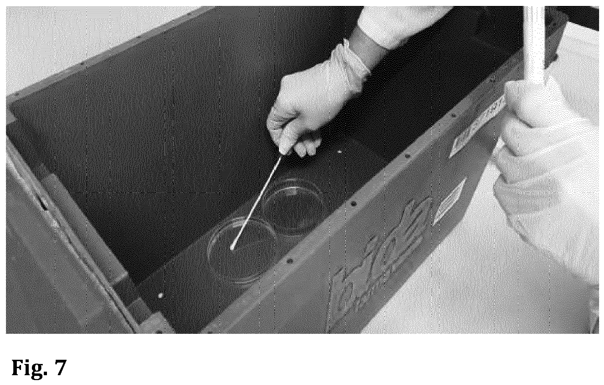

Figures 6 and 7 show samples being collected from the inoculated surfaces of the petri dishes of figure 5.

### DESCRIPTION OF A MANNER TO CARRY OUT THE INVENTION

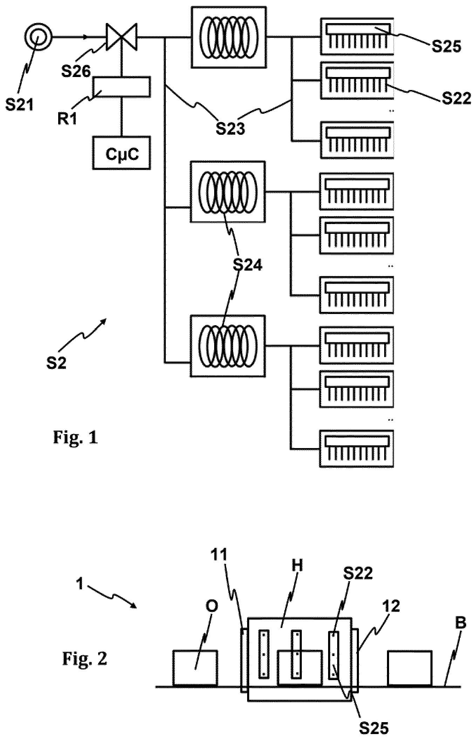

As schematically shown in Figure 2, the invention relates to an apparatus1 for exposing objects O to a disinfecting heat treatment. The heat treatment is performed in a chamber H provided with an inlet 11 and an outlet 12, both intended for allowing the passage of a conveyor belt B (the apparatus can be equipped with rollers, driven by a motor or not, or can be envisaged for being coupled directly to a pre-existing conveyor belt B) for conveying the objects O and passing them through the chamber H, a heat distribution system inside the chamber H. The inlet and the outlet will preferably be provided with insulating curtains to prevent heat from leaking out and to help keep the internal temperature uniform. Another non-illustrated possibility is the incorporation of gates and/or intermediate compartments for reducing thermal gradients and minimising heat loss.

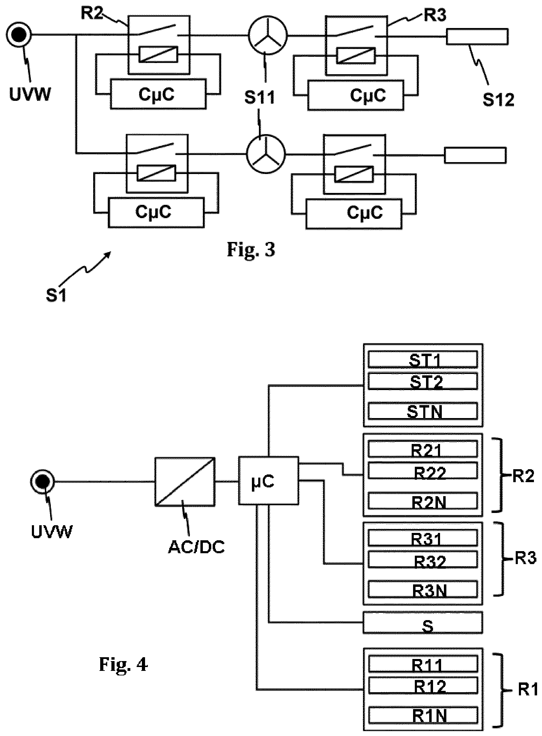

In particular, according to the invention, the heat distribution system comprises:
- an air convection subsystem S1 for maintaining a first minimum temperature T1 in the chamber H, as depicted in Figure 3; and
- a compressed air injection subsystem S2 for injecting compressed air at a second temperature T2, as shown in Figure 1.

As explained above, the subsystems S1, S2 are configured such that the second temperature T2 is higher than the first temperature T1. In other words, according to the invention, an essentially uniform minimum temperature in the chamber H is ensured as a result of the first subsystem, whereas the second subsystem is intended for locally increasing on the surface of the object O above the convection temperature and for a short time interval (i.e., the time interval that can be tolerated by the object O according to its nature), to ensure complete disinfection.

As shown in Figures 1 to 3:
- the first subsystem S1 comprises at least one fan S11 and a resistor S12;
- the second subsystem S2 comprises a compressed air source S21 and air injection nozzles S22 connected to the source S21 by means of a distribution circuit S23, in the illustrated embodiments by means of collectors S25, the circuit S23 being provided with electrical resistors S24 for heating the air to the second temperature T2.

The nozzles S22 can be arranged in many ways, although the arrangement of nozzles which produce an air blade or curtain is preferred. They can also be arranged such that the object O must pass through several curtains, for example, three as shown in Figure 2.

Compressed air can be provided by means of a pressurised tank or also by means of a compressor or turbines, using air from the surroundings, such that gas cylinders can also be dispensed with.

To control exposure time to the injected hot air, a valve S26, preferably a solenoid valve S26, arranged between the compressed air S21 source S21 and the air heating means S24 is contemplated. The temporal injected air pulse profiles can be adjusted to the conditions of the objects O, and particularly to the composition or content thereof.

According to another advantageous feature, the apparatus comprises one or several temperature sensors for detecting the temperature of the injected air and/or of the chamber ST1, ST2, STN, intended for allowing temperature control, and for ensuring that the desired temperature ranges are reached, and thereby preventing treatments with temperatures below the minimum temperature for eliminating viruses or pathogens.

According to a particularly advantageous feature, the apparatus comprises an array of identification or counting sensors (code reader) for controlling the object (ID of the object, type of object, etc.) and/or the number of disinfected objects while at the same time controlling the exit of the disinfected objects from the inside of the chamber H.

Each pathogen will have a different elimination temperature, but it is preferable that the first subsystem S1 is sized to maintain a minimum temperature of 80 °C in the chamber H, and the second subsystem S2 is configured for increasing the temperature of the air coming from the compressed air source S21 to a minimum temperature 140 °C, where it can reach up to 400 °C. The inventors were able to prove that this combination is suitable for achieving complete elimination of viruses or pathogens.

As illustrated by means of Figure 4, the apparatus comprises a controller µC for controlling fan control relays R2, heating resistors R3 and valves R1, through connections CµC with the controller. The controller can also have Ethernet connectivity, Wi-Fi connectivity, Bluetooth connectivity, etc.

In figures 3 and 4, UVW represents a three-phase electrical power source. The reference symbol S represents a screen.

The apparatus has been described in the context of a continuous inspection line. Evidently, it could also operate in a stationary manner, i.e., by introducing the objects in batches such that they remain still in the interior while being sterilised. In this case, it can be contemplated that the inlet 11 and the outlet 12 are the same opening.

The apparatus of the invention has been subjected to a series of microbiological tests to assess its efficacy in disinfecting objects. The methods and results of these tests will be presented below.

### Anti-Bacterial Effectiveness:

### Method:

First, an analysis of the anti-bacterial effectiveness of the apparatus was carried out. The experimental method included use of the apparatus of the invention in real time by applying it to plastic cuvettes with petri plates containing the following bacterial strains:
- *Staphylococcus aureus subsp. Aureus Rosenbach 1884 CECT 435*
- Bacillus subtilis subsp. Spizizenii Nakamura et al. 1999 CECT 356
- Listeria monocytogenes (Murray et al. 1926) Pirie 1940 CECT 935

Each of the strains was inoculated at a bacterial count of 10⁴-10⁵ cfu/100 cm² of surface area.

Inoculation was carried out at two points of each cuvette:
- 1^{st} point: cuvette floor (100 cm²)
- 2^{nd} point: cuvette wall (100 cm²)

Each point was analysed before the thermal treatment (to ascertain the initial bacterial load), after the continuous treatment and 1 second after the treatment.

### Results:

### - STRAIN: Staphylococcus aureus

**Continuous Treatment:**

| **Test Samples** | **Cuvette Wall (cfu/cm²)** | **Cuvette Floor (cfu/cm²)** |
|---|---|---|
| **Control Sample** (before continuous treatment) | 33,000 | 51,000 |
| **After Continuous Treatment** | 11,000 | 36,000 |

**Treatment with 1 second pause:**

| **Test Samples** | **Cuvette Wall (cfu/cm²)** | **Cuvette Floor (cfu/cm²)** |
|---|---|---|
| **Control Sample** (before treatment with 1 second pause) | 46,000 | 76,000 |
| **After Treatment with 1 second pause** | 2,100 | 15,000 |

With respect to the initial inoculation of the cuvette floor with 51,000 cfu, the continuous treatment resulted in a bacterial count of 36,000 cfu of the tested bacteria *Staphylococcus aureus.*

With respect to the initial inoculation of the cuvette wall with 33,000 cfu, the continuous treatment resulted in a bacterial count of 11,000 cfu of the tested bacteria *Staphylococcus aureus.*

With respect to the initial inoculation of the cuvette floor with 76,000 cfu, the treatment with a 1 second pause resulted in a bacterial count of 15,000 cfu of the tested bacteria *Staphylococcus aureus.*

With respect to the initial inoculation of the cuvette wall with 46,000 cfu, the treatment with a 1 second pause resulted in a bacterial count of 2,100 cfu of the tested bacteria *Staphylococcus aureus.*

### - STRAIN: Bacillus subtilis

**Continuous Treatment:**

| **Test Samples** | **Cuvette Wall (cfu/cm²)** | **Cuvette Floor (cfu/cm²)** |
|---|---|---|
| **Control Sample** (before continuous treatment) | 35,000 | 56,000 |
| **After Continuous Treatment** | 15,000 | 27,000 |

**Treatment with 1 second pause:**

| **Test Samples** | **Cuvette Wall (cfu/cm²)** | **Cuvette Floor (cfu/cm²)** |
|---|---|---|
| **Control Sample** (before treatment with 1 second pause) | 48,000 | 62,000 |
| **After Treatment with 1 second pause** | 2,500 | 14,000 |

With respect to the initial inoculation of the cuvette floor with 56,000 cfu, the continuous treatment resulted in a bacterial count of 27,000 cfu of the tested bacteria *Bacillus subtilis.*

With respect to the initial inoculation of the cuvette wall with 35,000 cfu, the continuous treatment resulted in a bacterial count of 15,000 cfu of the tested bacteria *Bacillus subtilis.*

With respect to the initial inoculation of the cuvette floor with 62,000 cfu, the treatment with a 1 second pause resulted in a bacterial count of 14,000 cfu of the tested bacteria *Bacillus subtilis.*

With respect to the initial inoculation of the cuvette wall with 48,000 cfu, the treatment with a 1 second pause resulted in a bacterial count of 2,500 cfu of the tested bacteria *Bacillus subtilis.*

### - STRAIN: Listeria monocytogenes

**Continuous Treatment:**

| **Test Samples** | **Cuvette Wall (cfu/cm²)** | **Cuvette Floor (cfu/cm²)** |
|---|---|---|
| **Control Sample** (before continuous treatment) | 36,000 | 34,000 |
| **After Continuous Treatment** | 3,900 | 11,000 |

**Treatment with 1 second pause:**

| **Test Samples** | **Cuvette Wall (cfu/cm²)** | **Cuvette Floor (cfu/cm²)** |
|---|---|---|
| **Control Sample** (before treatment with 1 second pause) | 27,000 | 55,000 |
| **After Treatment with 1 second pause** | 480 | 900 |

With respect to the initial inoculation of the cuvette floor with 34,000 cfu, the continuous treatment resulted in a bacterial count of 11,000 cfu of the tested bacteria *Listeria monocytogenes.*

With respect to the initial inoculation of the cuvette wall with 36,000 cfu, the continuous treatment resulted in a bacterial count of 3,900 cfu of the tested bacteria *Listeria monocytogenes.*

With respect to the initial inoculation of the cuvette floor with 55,000 cfu, the treatment with a 1 second pause resulted in a bacterial count of 900 cfu of the tested bacteria *Listeria monocytogenes.*

With respect to the initial inoculation of the cuvette wall with 27,000 cfu, the treatment with a 1 second pause resulted in a bacterial count of 480 cfu of the tested bacteria *Listeria monocytogenes.*

The above results can be summarized as follows:

| **STRAIN** | **Reduction Achieved (cfu/cm²)** |
|---|---|
| *Staphylococcus aureus* | 15,000 cfu |
| *Bacillus subtillis* | 20,000 cfu |
| *Listeria monocytogenes* | 23,000 cfu |

In addition, the result of the test for COVID-19 by PCR has been negative after the heat treatment in real time. Therefore, the presence of COVID-19 is not detected in the analyzed samples.

### Anti-Viral Effectiveness:

### Method:

The experimental method included use of the apparatus of the invention in real time with petri dishes containing the following viral strain: Quantitative Synthetic SARS-CoV-2 RNA, VR-3276SD (portions of ORF 1ab, E, N genes).

The strain of SARS-CoV-2 was inoculated onto two petri dishes and placed on the floor of a plastic crate. A sample was taken from each inoculated surface before the treatment to confirm the presence of virus on the surface and another sample was taken after the treatment.

The analysis has demonstrated the inactivation of the tested strain SARS-CoV-2 RNA, VR-3276SD using the apparatus of the invention.

### Results:

**PCR Results:**

| **(PCR) Test Samples** | **Results** |
|---|---|
| Control - extraction (process blank) | Not detected |
| Control + Master Mix | Detected |
| Control - Master Mix | Not detected |
| **Surface sample before treatment (before continuous treatment)** | **Detected/sample** |

| **(PCR) Test Samples** | **Results** |
|---|---|
| Control - extraction (process blank) | Not detected |
| Control + Master Mix | Detected |
| Control - Master Mix | Not detected |
| **Surface sample after treatment (before continuous treatment)** | **Not detected/sample** |

To summarise the above results: with respect to the initial sample inoculated on a Petri dish and placed at the bottom of the plastic crate (before treatment), the presence of the strain Quantitative Synthetic SARS-CoV-2 RNA, VR-3276SD could be detected.

After the treatment, presence of the strain Quantitative Synthetic SARS-CoV-2 RNA, VR-3276SD could not be detected on the inoculated sample surface.

In this text, the term "comprises" and its derivations such as "comprising", etc. must not be understood in an exclusive sense, i.e., these terms must not be interpreted as excluding the possibility of what is described and defined may include further elements, steps, etc.

The invention is obviously not limited to the specific embodiment(s) that have been described, rather it also covers any variation that may be considered by any person skilled in the art (for example, in relation to the choice of materials, dimensions, components, configuration, etc.), within the general scope of the invention as defined in the claims.

## Claims

1. An apparatus (1) for exposing objects (O) to a disinfecting heat treatment, the apparatus (1) comprising a chamber (H) provided with an inlet (11) and an outlet (12), both intended for allowing the passage of a conveyor belt (B) for conveying the objects (O) and passing them through the chamber (H), a heat distribution system inside the chamber (H), **characterised in that** the heat distribution system comprises:
- an air convection subsystem (S1) for maintaining a first minimum temperature (T1) in the chamber (H);
- a compressed air injection subsystem (S2) for injecting compressed air at a second temperature (T2);
the subsystems (S1, S2) being configured such that the second temperature (T2) is higher than the first temperature (T1).

2. The apparatus according to claim 1, wherein:
- the first subsystem (S1) comprises at least one fan (S11) and a resistor (S12);
- the second subsystem (S2) comprises a compressed air source (S21) and air injection nozzles (S22) connected to the source (S21) by means of a distribution circuit (S23), the circuit (S23) being provided with electrical resistors (S24) for heating the air to the second temperature (T2).

3. The apparatus according to claim 2, wherein the second subsystem (S2) comprises collectors (S25) each provided with several nozzles (S22).

4. The apparatus according to claim 2 or 3, wherein the nozzles (S22) are arranged linearly such that they produce an air curtain.

5. The apparatus according to claim 2, 3 or 4, comprising a valve (S26), preferably a solenoid valve (S26), arranged between the compressed air (S21) source (S21) and the air heating means (S24).

6. The apparatus according to any of the preceding claims, comprising one or several temperature sensors for detecting the temperature of the injected air and/or of the chamber (ST1, ST2, STN).

7. The apparatus according to any of the preceding claims, comprising a code reader arranged before the inlet (11), such that the objects (O) can be identified and characterised to adjust the air heating parameters.

8. The apparatus according to any of the preceding claims, wherein the first subsystem (S1) is sized to maintain a minimum temperature of 80 °C in the chamber (H).

9. The apparatus according to any of the preceding claims, wherein the second subsystem (S2) is configured for increasing the temperature of the air coming from the compressed air source (S21) to a temperature comprised higher than 140 °C.

10. The apparatus according to any of the preceding claims, wherein the air from the compressed air source (S21) is at a pressure comprised between 4 and 6 bar, and is preferably 5 bar.

11. The apparatus according to any of the preceding claims, comprising insulating curtains in the inlet (11) and in the outlet (12) of the chamber (H).

12. The apparatus according to claim 11 wherein the insulating curtains are movable Teflon belts.

13. The apparatus according to any of the preceding claims, comprising a controller (µC) for controlling fan control relays (R2), heating resistors (R3) and valves (R1).

14. The apparatus according to any of the preceding claims, comprising a screen (S) for displaying the operation of the invention, the configuration of the parameters, or information about the treated objects (O), the screen (S) being preferably configured to display the disinfection rate and/or the number of disinfected objects (O).

15. Method for disinfecting objects, which consists in introducing an object in an apparatus according to any of the previous claims, wherein the hot air is continuous or pulsed.
